(19) **Europäisches Patentamt**
**European Patent Office**
**Office européen des brevets**

(11) **EP 2 389 929 A1**

(12) **EUROPEAN PATENT APPLICATION**

(43) Date of publication:
**30.11.2011 Bulletin 2011/48**

(51) Int Cl.:
***A61K 9/20*** *(2006.01)* ***A61K 31/675*** *(2006.01)*

(21) Application number: **10164417.7**

(22) Date of filing: **30.05.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicant: **Abdi Ibrahim Ilac Sanayi ve Ticaret Anonim Sirketi Istanbul (TR)**

(72) Inventors:
• **Farshi, Farhad**
**34555, ISTANBUL (TR)**
• **Avci, Recep**
**34555, ISTANBUL (TR)**
• **Sahin, Sinem**
**34555, ISTANBUL (TR)**
• **Soylemez, Serdar**
**34555, ISTANBUL (TR)**

(54) **Pharmaceutical formulations of tenofovir**

(57) This invention is related to tenofovir or pharmaceutically acceptable salts thereof formulations having pregelatinized starch prepared by direct compression which provides good compressibility, flowability and good chemical stability.

EP 2 389 929 A1

## Description

Technical Field

This invention is related to tenofovir or pharmaceutically acceptable salts thereof formulations having pregelatinized starch prepared by direct compression which provides good compressibility, flowability and good chemical stability.

## Background Art

Human immunodeficiency virus (HIV) infection and related diseases are a major public health problem worldwide. Since its initial recognition in the early 1980's, AIDS has spread rapidly and has now reached epidemic proportions within a relatively limited segment of the population. AIDS causes a gradual breakdown of the body's immune system as well as progressive deterioration of the central and peripheral nervous systems. Intensive research has led to the discovery of the responsible agent, now more commonly referred to as the human immunodeficiency virus.

HIV is a member of the class of viruses known as retroviruses. The retroviral genome is composed of RNA which is converted to DNA by reverse transcription. This retroviral DNA is then stably integrated into a host cell's chromosome and, employing the replicative processes of the host cells, produces new retroviral particles and advances the infection to other cells. HTV appears to have a particular affinity for the human T-4 lymphocyte cell which plays a vital role in the body's immune system. HIV infection of these white blood cells depletes this white cell population. The immune system is rendered inoperative and ineffective against various opportunistic diseases. As a result the immune system can no longer fight off other infections that it would usually be able to prevent.

When HIV infects a cell, reverse transcriptase copies the viral single stranded RNA genome into a double-stranded viral DNA. The viral DNA is then integrated into the host chromosomal DNA which then allows host cellular processes, such as transcription and translation to reproduce the virus. As HIV uses reverse transcriptase to copy its genetic material and generate new viruses, specific drugs have been designed to disrupt the process and thereby suppress its growth. RTis block reverse transcriptase's enzymatic function and prevent completion of synthesis of the double-stranded viral DNA thus preventing HIV from multiplying.

Collectively, these drugs are known as reverse transcriptase inhibitors and include the nucleoside and nucleotide analogues zidovudine (trade name Retrovir), lamivudine (Epivir) and tenofovir (Viread), as well as non-nucleoside inhibitors, such as nevirapine (Viramune).

Tenofovir belongs to a class of antiretroviral drugs known as nucleotide analogue reverse transcriptase inhibitors (NtRTIs), which block reverse transcriptase, an enzyme crucial to viral production in HIV infected people.

Tenofovir disoproxil fumarate [tenofovir DF, formerly known as bis(POC)-PMPA] is a bis-ester prodrug of the acyclic nucleoside phosphonate tenofovir.

Tenofovir disoproxil fumarate (a prodrug of tenofovir) is a fumaric acid salt of bis-isopropoxycarbonyloxymethyl ester derivative of tenofovir.The chemical name of tenofovir disoproxil fumarate is 9-[(R)-2-[[bis[[(isopropoxycarbonyl) oxy]methoxy]phosphinyl]methoxy]propy I]adenine fumarate (1:1).

In vivo tenofovir disoproxil fumarate is converted to tenofovir, an acyclic nucleoside phosphonate (nucleotide) analog of adenosine 5'-monophosphate.

Tenofovir disoproxil fumarate which is the first nucleotide analog approved for HIV-1 treatment, remains in cells for longer periods of time than many other antiretroviral drugs, thereby allowing for once-daily dosing.

It is sold under the brand name VIREAD® as tablets are for oral administration, each tablet containing 300 mg of tenofovir disoproxil fumarate, which is equivalent to 245 mg of tenofovir disoproxil. In vivo, tenofovir disoproxil fumarate is converted to tenofovir, an acyclic nucleoside phosphonate (nucleotide) analog of adenosine 5'-monophosphate.

Tenofovir DF is firstly disclosed by WO 99/05150 A (GILEAD SCIENCES INC) 04.02.1999 .It discloses a solid pharmaceutical composition comprising tenofovir or pharmaceutically acceptable salt ,wherein pregelatinized starch is used in 5% (w/w) of tablet core in a wet granulation method.

Summary of invention

It is an objection of present invention to provide a pharmaceutical composition comprising tenofovir or its pharmaceutically acceptable salt and a specific amount of pregelatinized starch having proper chemical stability, good compressibility, flowability and no sticking to the dies or punches of tablet press by direct compression.

## Technical Problem

In pharmaceutical technology, the chemical stability of active ingredient is a concern to minimize the generation of impurities and ensure adequate shelf-life. It is influenced not only by molecular structure but also by many parameters. Factors determining stability of drug substance include factors such as light/moisture/temperature sensivity, pH and other excipients and process design. The production method should be properly chosen to overcome any potential stability, compressibility and bioavailability problems.

Tenofovir Disoproxil Fumarate is known to be unstable or at least has a reduced stability. Tenofovir disoproxil fumarate has a low $pK_a$ value which is indicative of the potential to cause acidic hydrolysis of active ingredients.

EMEA (European Medicines Agency) declares that the reference product (VIREAD®) which is produced by wet granulation has stability problem. It is clearly declared that the stability data provided do not yet support the claimed limit of %8 for impurities/degradation products in the shelf-life specification.

In pharmaceutical technology, although wet granulation is widely used, it is also well known that wetting and drying process of wet granulation affects stability of the product. This method may not be appropriate for thermolabile or light sensitive active ingredient. Furthermore the production can not be reproducible regarding to climatic conditions. Expensive and specialized equipment, labor, more processing time, power are required.

Solution to Problem

In order to prevent the stability problem in case of thermolabile and light sensitive of drug, a heat and moisture free process usually is preferred. Direct compression is obviously the most advantageous process, due to no pre treatment of the powder blend procedure is required. Because of direct compression involves only blending and compression, offers advantages particularly in terms of stability as well as production.

Thus manufacturers would prefer to use direct compression techniques since it is advantageous over granulation. Direct compression technique has quick processing and cost advantages.

Direct compression provides technical benefits as well as economic benefits. Direct compression is a preferred process for its simplicity and economy saving as well. Less number of equipments, reduced labor and less processing time and power are required.

Furthermore, dissolution rate is improved by utilization of direct compression. Direct compressed tablet integrates quickly to smaller particles which have an increased surface area for dissolution.

Through a simpler process, process validation is easier and batch-to-batch variation is negligible when compared with other techniques.

It is invented that by direct compression pharmaceutical compositions of tenofovir disoproxil fumarate having proper chemical stability, eligible granule friability, tablet friability, hardness, disintegration rate and drug dissolution rate.

In other aspect of this invention, the composition comprises starch, especially pregelatinized starch. Pregelatinized starch is used in an optimum amount that starch simply breaks the tablet apart to expose the drug substance for dissolution.

**Description of embodiments**

In the present invention, inventors provide a pharmaceutical formulation and a production method to get a better stability property by a simpler manufacturing method.

According to this invention, the pharmaceutical compositions of tenofovir or pharmaceutically acceptable salts thereof, especially tenofovir disoproxil fumarate can be prepared by direct compression, may further comprise pharmaceutically acceptable excipients or excipients selected from the group consisting of diluents, binders, disintegrants, fillers, lubricants, glidants, mixtures thereof and the like.

The term 'filler' and the term 'diluent' are herein used interchangeably. Fillers fill out the size of a composition, making it practical to produce and convenient for the consumer to use. Suitable filler/diluent includes, but are not limited, to calcium carbonate, calcium phosphate, dibasic calcium phosphate, tribasic calcium sulfate, calcium carboxymethylcellulose, cellulose, dextrin derivatives, dextrin, dextrose, fructose, lactitol, lactose lactose (e.g. spray-dried lactose, α-lactose, β-lactose, Tablettose®, various grades of Pharmatose®, Microtose® or Fast-Floc®), methylcellulose polymers such as, e.g., Methocel A®, Methocel A4C®, Methocel A 15C®, Metocel A4M®), hydroxyethylcellulose, hydroxypropylcellulose, L-hydroxypropylycellulose (low substituted), hydroxypropyl methylcellulose (HPMC) (e.g. Methocel E®, F and K, Metolose SH® of Shin-Etsu, grades of Methocel F® and Metolose 65 SH®, the 4,000, 15,000 and 100,000 cps grades of Methocel K®; and the 4,000, 15,000, 39,000 and 100,000 grades of Metolose 90 SH®), sodium carboxymethylcellulose, carboxymethylene, carboxymethylhydroxyethylcellulose and other cellulose derivatives, starches or modified starches ( including potato starch, wheat starch, corn starch, rice starch, pregelatinized maize starch), magnesium carbonate, magnesium oxide, maltitol, maltodextrins, maltose, sorbitol, starch, sucrose, sugar, and xylitol, erythritol.

A disintegrant is a substance which helps the composition break up once ingested. Disintegrants are, but not limited to, cross linked polyvinylpyrolidone (crospovidone, polyplyplasdone XL®, kollidon CL®); starches such as maize starch and dried sodium starch glycolate; gums such as maize starch and dried sodium starch glycolate; gums such as alginic acid, sodium alginate, guar gum; croscarmellose sodium; cellulose products such as microcrystalline cellulose and its salts, microfine cellulose, low-substituted hydroxypropylcellulose, mixtures thereof and the like.

Glidants improve the flowability of the composition. The composition may also comprise a glidant. Glidants are, but not limited to, colloidal silica, silicon dioxide,powdered cellulose, talc, tribasic calcium phosphate, mixtures thereof and the like. Preferably, pregelatinized starch is used as disintegrating agent. An additional disintegrating agent can be used.

The presence of a lubricant is particularly preferred when the composition is a tablet as lubricants to improve the tabletting process. Lubricants prevent composition ingredients from clumping together and from sticking to the tablet punches or capsule filling machine and improve flowability of the composition mixture. Lubricants are, but not limited to sodium oleate, sodium stearate, sodium benzoate, sodium stearate, sodium chloride, stearic acid, sodium stearyl fumarate, calcium stearate, magnesium stearate, magnesium lauryl sulfate, sodium stearyl fumarate, sucrose esters or fatty acid, zinc, polyethylene glycol, talc, mixtures thereof and the like.

One or more these additives can be selected and used by the artisan having regard to the particular desired properties of the solid dosage form. The amount of each type of additive employed, e.g. glidant, binder, disintegrant, filler or diluent and lubricant may vary within ranges conventional in the art.

Suitable pharmaceutical compositions include, but are not limited to, capsules, tablets, granules, powders and unit dose pockets. Preferably oral pharmaceutical formulation is tablet. The tablet can be coated or non-coated.

According to a preferred embodiment of the present invention, the tablet cores are coated. Coating agents are, but not limited to, sugars, hydroxypropyl methylcellulose, hydroxypropyl cellulose, methylcellulose, ethylcellulose, polyvinyl alcohol, sodium carboxylmethylcellulose, coatings based on methacrylic acid and its esters, such as Eudragit ®, mixtures thereof and the like. Coating may further contain an excipient or excipients, for example, titanium dioxide, talc, ferric oxide, polyethylene glycol and the like. As a coating agent multifunction ingredient such as Opadry® can also be used.

In other aspect of this invention, the composition comprises starches,especially pregelatinized starch. Pregelatinized starch is a directly compressible form of starch consisting of intact and partially hydrolyzed ruptured starch grains.

Pregelatinized starch has multiple uses in formulations; as a binder, filler and disintegrant. The principal application of pregelatinized starch in tablet formulation is as a disintegrating agent. In aspect of this invention, functional denomination is not important.

In addition to known starch properties, pregelatinized starch has additional benefits when formulating with a moisture sensitive active. Pregelatinized starch has been shown to exhibit a lower propensity for moisture, thus providing excellent stabilization in formulations with moisture-sensitive active. Pregelatinized starch absorbs moisture from the atmosphere, or moisture introduced by other raw materials, the starch binds this moisture and makes it unavailable for unwanted chemical reactions. In addition to this, it is well known that pregelatinized starch shows improved flowability and lubricity while retaining its disintegrant capability and moisture stability.

Another aspect of this invention is related to effects of pregelatinized starch which is used as a disintegrating agent. In tablet formulation pregelatinized starch is used is in a specific range by the weight of the composition. As a disintegrant, pregelatinized starch displays its best disintegrating properties when said excipient is in 5-15 %( w /w) of total tablet weight. Dissolution in formulation containing less than 5 % (w/w) of pregelatinized starch is insufficient. In formulation containing more than15 % (w /w) of pregelatinized starch, sticking problem occurs and proper production becomes impossible.

In yet another aspect of this invention is a pharmaceutical composition comprising tenofovir or pharmaceutically acceptable salt and 5 % -15% (w/w) of pregelatinized starch prepared by direct compression. Pregelatinized starch in an amount of 5%-15% (w /w) of total tablet weight is found to be sufficient for dissolution and necessary amount for proper tablet pressing process. A similar content for pregelatinized starch in direct compression was not cited before.

On the other hand this invention includes a preparation method for direct compression of tenofovir disoproxil fumarate. This method comprising steps of a. tenofovir disoproxil fumarate, filler/diluent, disintegrant, glidant are sieved by using suitable milling equipment. b. The sieved powder is installed into container and mixed c. Pre-sieved lubricant is installed into container onto powder mixture prepared at step b and mixed. d. The final mixture is compressed e. a coating agent or mixtures of coating agents are added into purified water and stirred. f. Core tablets are installed into a coating pan and coated with the coating suspension prepared at step e.

Packaging of tenofovir or pharmaceutically acceptable salts thereof is another important step due to its big influence on stability. For thus at least the secondary packaging of such tablets have to be none light permeable. The other issue is the primary packaging which has to have at least the ability to protect the product against moisture. Possible primary packaging can vary from polyester fiber packing material to containers with desiccants or other packaging materials which can fulfill such requirements to have a stabile finished product.

In a embodiment of this invention, active ingredient is in the range of from about %10 to about 70%, diluent is in the range of from about %20 to about 60%, disintegrant is in the range of from about 7% to about 20%, pregelatinized starch is in the range of from about 1% to about 20%, glidant is in the range of from about 0.01 % to about 3%, lubricant is in the range of from about 0.1% to about 10%, coating agent is in the range of from about 0.1 % to about 10% by weight of total pharmaceutical composition.

Preferably, pharmaceutical composition comprising tenofovir disoproxil fumarate thereof is 41.7%, filler/diluent is 38.90 %, disintegrant is 12.0%, pregelatinized starch is 5%, glidant is 0.2%, lubricant is 4.5%, coating agent is 2.7% by the weight of total pharmaceutical composition (Table 1).

In yet another aspect of this invention is a tablet of tenofovir or its pharmaceutically acceptable salt thereof characterizing in that the pharmaceutical composition has an in vitro dissolution profile with a similarity factor (f2) of at least 50 to 100 compared to the reference (Viread® , Gilead Sciences, Inc) dissolution profile.

The similarity factor f2 is a measurement of the similarity as shown in equation 1.

**(Equation 1- Similarity Factor)**

$$f_2 = 50 * \log \frac{100}{\sqrt{1 + \frac{1}{n}\sum_{t=1}^{n}(R_t - T_t)^2}}$$

n: is the number of sampling time points

$R_t$ : is the amount drug released from a reference batch at time t

$T_t$: is the amount drug released from a testbatch at time t.

Generally, f2 values greater than 50 ensure sameness of the performance of the reference product and test product.

Example

**Table 1**

| Total Pharmaceutical Composition | | |
|---|---|---|
| Ingredients | mg | % |
| Tenofovir Disoproxil Fumarate | 300.00 | 41.70 |
| Filler/Diluent | 279.76 | 38.90 |
| Disintegrant | 50.4 | 7.00 |
| Pregelatinized Starch | 36.00 | 5.00 |
| Glidant | 1.44 | 0.20 |
| Lubricant | 32.40 | 4.50 |
| Coating Agent | 20.00 | 2.70 |
| **Total** | **720.00** | **100.00** |

Tenofovir Disoproxil Fumarate test tablet is released in 0.1 N HCl environment under conditions of 900 ml of a dissolution medium at 37°C $\pm$0.5°C, USP method-II (pedal), 50 rpm speed wherein tablet exhibits a dissolution profile (Table 2). F2 value is 91.6.Comparison of dissolution profiles of the test and reference tenofovir disoproxil fumarate tablet showed clearly that f2 values are proper in 0.1 N HCl, pH=6.8, pH=4.5 media which are known to be a stimulation for gastric fluid.

**Table 2**

| Time (Minutes) | Dissolved % | |
|---|---|---|
| | Viread® Gilead Sciences, Inc. (Reference) | Tenofovir Disoproxil Fumarate Film Tablet (Test) |
| 10 | 96,1 | 95,6 |

(continued)

| Time (Minutes) | Dissolved % | |
| --- | --- | --- |
| | **Viread® Gilead Sciences, Inc. (Reference)** | **Tenofovir Disoproxil Fumarate Film Tablet (Test)** |
| 15 | 97,6 | 98,6 |
| 20 | 98,1 | 99,3 |
| 30 | 98,4 | 99,7 |
| 45 | 98,8 | 99,9 |

**Claims**

1. A pharmaceutical composition of tenofovir or pharmaceutically acceptable salts thereof **characterized in that** a) it is prepared by direct compression and b) it includes starch or mixtures of starches in the range of 5%-15% by total weight.

2. As claimed in claim 1, starch is pregelatinized starch.

3. According to claim 1, the pharmaceutical composition, comprising :tenofovir or pharmaceutically acceptable salts thereof is in the range of from 10% to 70%,diluent is in the range of from %20 to 60%, disintegrant is in the range of from 7% to 20%, pregelatinized starch is in the range of from about 1% to about 20%, glidant is in the range of from 0.01% to 3%, lubricant is in the range of from 0.1% to 10%, coating agent is in the range of from 0.1% to 10% by weight of total pharmaceutical composition.

4. According to claim 3, pharmaceutical composition comprising tenofovir or pharmaceutically acceptable salts thereof is 41.7%, filler/diluent is 38.90 %, disintegrant is 12.0%, pregelatinized starch is 5%, glidant is 0.2%, lubricant is 4.5%, coating agent is 2.7% by weight of total pharmaceutical composition.

5. According to claim 1, pharmaceutical composition is prepared by the following steps: a. tenofovir or pharmaceutically acceptable salts thereof , filler/diluent, disintegrant, pregelatinized starch, glidant are sieved by using suitable milling equipment. b. The sieved powder is installed into container and mixed c. Pre-sieved lubricant is installed into container onto powder mixture prepared at step b and mixed. d. The final mixture is compressed e. a coating agent or mixtures of coating agents are added into purified water and stirred. f. Core tablets are installed into a coating pan and coated with the coating suspension prepared at step e.

6. According to claim 1, a tablet of tenofovir or a pharmaceutically acceptable salt thereof has an in vitro dissolution profile with a similarity factor (f2) of at least 50 when compared to the dissolution profile of reference (Viread® , Gilead Sciences, Inc) product.

7. According to claim 6, in dissolution conditions of tenofovir or pharmaceutically acceptable salts thereof are 0.1 N HCl, 900 ml of a dissolution medium at 37°C $\pm$0.5°C and USP method-II (pedal) and 50 rpm speed, f2 is 91.6.

8. According to preceding claims, pharmaceutically acceptaple salt of tenofovir is disoproxil fumarate .

Europäisches
Patentamt

European
Patent Office

Office européen
des brevets

**EUROPEAN SEARCH REPORT**

Application Number

EP 10 16 4417

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 99/05150 A1 (GILEAD SCIENCES INC [US]; MUNGER JOHN D JR [US]; ROHLOFF JOHN C [US];) 4 February 1999 (1999-02-04) * page 8, line 24 - line 37; example 4 * | 1-8 | INV. A61K9/20 A61K31/675 |
| X | EP 1 923 063 A2 (GILEAD SCIENCES INC [US]) 21 May 2008 (2008-05-21) * paragraph [0103] - paragraph [0110] * | 1-8 | |
| X | WO 2006/017044 A2 (GILEAD SCIENCES INC [US]; DAHL TERRENCE C [US]) 16 February 2006 (2006-02-16) * formulation A * * abstract * | 1-8 | |
| X | WO 2007/068934 A2 (CIPLA LTD [IN]; CURTIS PHILIP ANTHONY [GB]; LULLA AMAR [IN]; MALHOTRA) 21 June 2007 (2007-06-21) * examples 1,2 * * page 13, line 22 - line 26 * | 1-8 | TECHNICAL FIELDS SEARCHED (IPC) A61K |
| X | WO 2008/140302 A1 (ULTIMORPHIX TECHNOLOGIES B V [NL]; DOVA EVANTHIA [NL]; MAZUREK JAROSLA) 20 November 2008 (2008-11-20) * page 43, line 31 - line 39 * * page 48, line 34 - line 41 * | 1-8 | |

-/--

The present search report has been drawn up for all claims

| Place of search | Date of completion of the search | Examiner |
|---|---|---|
| Munich | 10 August 2010 | Sproll, Susanne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 10 16 4417

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TE WIERIK G H P ET AL: "A new generation of starch products as excipient in pharmaceutical tablets. II. High surface area retrograded pregelatinized potato starch products in sustained-release tablets" JOURNAL OF CONTROLLED RELEASE, ELSEVIER, AMSTERDAM, NL LNKD- DOI:10.1016/S0168-3659(96)01535-0, vol. 45, no. 1, 3 March 1997 (1997-03-03), pages 25-33, XP004524228 ISSN: 0168-3659 * the whole document * | 1-8 | |

-----

TECHNICAL FIELDS
SEARCHED      (IPC)

The present search report has been drawn up for all claims

| Place of search | Date of completion of the search | Examiner |
|---|---|---|
| Munich | 10 August 2010 | Sproll, Susanne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 10 16 4417

This annex lists the patent family members relating to the patent documents cited in the above-mentioned European search report.
The members are as contained in the European Patent Office EDP file on
The European Patent Office is in no way liable for these particulars which are merely given for the purpose of information.

10-08-2010

| Patent document cited in search report | | Publication date | Patent family member(s) | | Publication date |
|---|---|---|---|---|---|
| WO 9905150 | A1 | 04-02-1999 | AT | 305009 T | 15-10-2005 |
| | | | AT | 288914 T | 15-02-2005 |
| | | | AT | 228526 T | 15-12-2002 |
| | | | AU | 756793 B2 | 23-01-2003 |
| | | | AU | 8582798 A | 16-02-1999 |
| | | | BR | 9811045 A | 22-08-2000 |
| | | | CA | 2298059 A1 | 04-02-1999 |
| | | | CN | 1264387 A | 23-08-2000 |
| | | | CN | 1554350 A | 15-12-2004 |
| | | | DE | 69809750 D1 | 09-01-2003 |
| | | | DE | 69809750 T2 | 21-08-2003 |
| | | | DE | 69829010 D1 | 17-03-2005 |
| | | | DE | 69829010 T2 | 13-04-2006 |
| | | | DE | 69831694 D1 | 27-10-2005 |
| | | | DE | 69831694 T2 | 13-07-2006 |
| | | | DK | 1243593 T3 | 14-11-2005 |
| | | | DK | 998480 T3 | 24-03-2003 |
| | | | EP | 0998480 A1 | 10-05-2000 |
| | | | ES | 2249511 T3 | 01-04-2006 |
| | | | ES | 2236389 T3 | 16-07-2005 |
| | | | ES | 2187989 T3 | 16-06-2003 |
| | | | HK | 1029793 A1 | 20-06-2003 |
| | | | HK | 1051373 A1 | 07-04-2006 |
| | | | HK | 1051360 A1 | 14-10-2005 |
| | | | ID | 24701 A | 03-08-2000 |
| | | | IN | 190780 A1 | 23-08-2003 |
| | | | JP | 4173202 B2 | 29-10-2008 |
| | | | JP | 2002511098 T | 09-04-2002 |
| | | | JP | 2008208126 A | 11-09-2008 |
| | | | KR | 20060038451 A | 03-05-2006 |
| | | | KR | 20060038452 A | 03-05-2006 |
| | | | NZ | 501287 A | 28-09-2001 |
| | | | PT | 1243590 E | 31-05-2005 |
| | | | PT | 998480 E | 31-03-2003 |
| | | | SG | 106656 A1 | 29-10-2004 |
| | | | SG | 106657 A1 | 29-10-2004 |
| | | | TW | 224103 B | 21-11-2004 |
| | | | TW | 242561 B | 01-11-2005 |
| EP 1923063 | A2 | 21-05-2008 | NONE | | |
| WO 2006017044 | A2 | 16-02-2006 | AR | 050424 A1 | 25-10-2006 |
| | | | AT | 447396 T | 15-11-2009 |
| | | | AU | 2005272032 A1 | 16-02-2006 |
| | | | BR | PI0513196 A | 29-04-2008 |
| | | | CA | 2570877 A1 | 16-02-2006 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.** EP 10 16 4417

This annex lists the patent family members relating to the patent documents cited in the above-mentioned European search report.
The members are as contained in the European Patent Office EDP file on
The European Patent Office is in no way liable for these particulars which are merely given for the purpose of information.

10-08-2010

| Patent document cited in search report | Publication date | Patent family member(s) | Publication date |
|---|---|---|---|
| WO 2006017044 A2 | | CN 1984640 A | 20-06-2007 |
| | | DK 1773296 T3 | 08-03-2010 |
| | | EA 200700311 A1 | 29-06-2007 |
| | | EP 1773296 A2 | 18-04-2007 |
| | | EP 2138162 A2 | 30-12-2009 |
| | | EP 2138163 A2 | 30-12-2009 |
| | | ES 2336017 T3 | 07-04-2010 |
| | | HK 1103026 A1 | 19-03-2010 |
| | | HR 20070040 A2 | 30-04-2007 |
| | | HR 20100038 T1 | 28-02-2010 |
| | | IS 8584 A | 21-12-2006 |
| | | JP 2008505902 T | 28-02-2008 |
| | | KR 20070041537 A | 18-04-2007 |
| | | PT 1773296 E | 04-02-2010 |
| | | SI 1773296 T1 | 30-04-2010 |
| | | US 2008035155 A1 | 14-02-2008 |
| | | ZA 200700932 A | 30-07-2008 |
| WO 2007068934 A2 | 21-06-2007 | AU 2006325404 A1 | 21-06-2007 |
| | | CA 2633603 A1 | 21-06-2007 |
| | | EP 2051703 A2 | 29-04-2009 |
| | | JP 2009519311 T | 14-05-2009 |
| | | KR 20080091767 A | 14-10-2008 |
| | | US 2008317852 A1 | 25-12-2008 |
| WO 2008140302 A1 | 20-11-2008 | NONE | |

EPO FORM P0459

**EP 2 389 929 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9905150 A